# FASCICULE DE BREVET EUROPEEN

(11) **EP 0 457 680 B1**
(45) Date de publication et mention de la délivrance du brevet: **07.12.1994**
(21) Numéro de dépôt: 91401257.0
(22) Date de dépôt: 15.05.1991
(51) Int. Cl.: C07D 211/70, C07K 15/12, A61K 31/15

(54) **Dérivés de l'oxime du 1,2,5,6-tétrahydropyridin-3-carboxaldéhyde, leur procédé de préparation et leur application comme médicaments**
Derivate des 1,2,5,6-Tetrahydropyridin-3-caboxaldehydoxims, Verfahren zu ihrer Herstellung und ihre Verwendung als Arzneimittel
Derivatives of 1,2,5,6-tetrahydropyridine-3-carboxaldehyde oxime, process for their preparation and their use as medicaments

(30) Priorité: 15.05.1990 IT 2031190
(43) Date de publication de la demande: 21.11.1991
(73) Titulaire: ROUSSEL-UCLAF, 75007 Paris (FR)
(72) Inventeur: Toja, Emilio, Milano (IT); Bonetti, Carla, Fontanella (Bergamo) (IT); Barzaghi, Fernando, I-20052 Monza-Milan (IT); Galliani, Giulio, 1 Monza (IT)
(74) Mandataire: Tonnellier, Marie-José

(56) Documents cités:
- EP-A- 0 271 798
- EP-A- 0 308 283
- EP-A- 0 308 284

## Description

La présente invention concerne de nouveaux dérivés de l'oxime du 1,2,5,6-tétrahydropyridin-3-carboxaldéhyde, leur procédé de préparation et leur application comme médicaments.

Le brevet EP A 308 283 décrit des dérivés de l'oxime du 1,2,5,6-tétrahydropyridin-3-carboxaldéhyde présentant une forte activité cholimétique centrale.

L'invention a pour objet les composés de formule (I) : 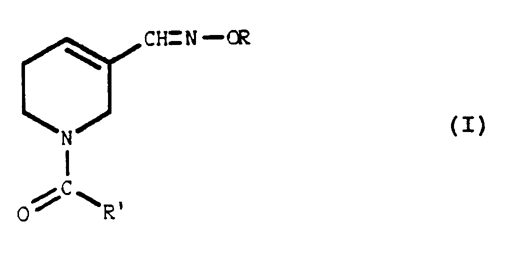
dans laquelle :
- R représente un atome d'hydrogène, un radical alkyle linéaire, ramifié ou cyclique, saturé ou insaturé renfermant jusqu'à 8 atomes de carbone,
- R' représente le reste d'un acide aminé ou d'un peptide renfermant 2, 3 ou 4 acides aminés, ainsi que leurs sels d'addition avec les acides organiques ou minéraux.

Dans la formule I, le groupement CO provient du groupement carboxyle alpha de l'acide aminé ou du groupement CO terminal du peptide.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléique, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

Lorsque R représente un radical alkyle saturé, linéaire ou ramifié, il s'agit de préférence d'un radical méthyle, éthyle, n-propyle, isopropyle, n-butyle, isobutyle, n-pentyle, tert-butyle, tert-pentyle, néopentyle ou n-hexyle.

Lorsque R représente un radical alkyle insaturé, il s'agit de préférence d'un radical éthylénique comme, par exemple, le radical vinyle, allyle, 1,1-diméthylallyle, 2-butényle, ou d'un radical acétylénique comme, par exemple, le radical éthynyle ou propynyle.

Lorsque R représente un radical alkyle cyclique, il s'agit de préférence d'un radical cyclopropyle, cyclobutyle, cyclopentyle, cyclohexyle, cyclopropylméthyle, cyclobutylméthyle, cyclopentylméthyle ou cyclohexylméthyle.

Dans la formule générale (I) et dans ce qui suit, l'acide aminé est un acide alpha-aminé et peut être choisi dans le groupe constitué par Ala, Val, Ival, Leu, Ile, Asp, Asn, Glu, Gln, Ser, Thr, Cys, Met Lys, Arg, Phe, Tyr, Trp, His et Pro, Nva, Nle, Hyp, Orn, ces acides étant sous la forme D ou L ainsi que par Sar et Gly ; dans le cas d'un peptide comprenant 2, 3 ou 4 acides aminés, ceux-ci sont choisis dans le groupe constitué par les acides aminés ci-dessus.

On admettra par convention que les symboles des acides alpha-amino carboxyliques représentent ces acides sous leur configuration D ou L ou par un mélange D et L, (par exemple, le terme Ala signifie Alanine sous forme D ou sous forme L, ou sous forme de mélange D et L).

L'invention a plus particulièrement pour objet :
- les composés de formule (I) dans lesquels R représente un radical alkyle linéaire, saturé ou insaturé renfermant jusqu'à 4 atomes de carbone,
- les composés de formule (I) dans lesquels R représente un radical méthyle,
- les composés de formule (I) dans lesquels R' représente le reste d'un acide alpha-amino carboxylique de configuration L,
- les composés de formule (I) dans lesquels R' représente le reste de l'alanine,
- les composés de formule (I) dans lesquels R' représente le reste d'un acide aminé renfermant deux fonctions acides, par exemple le reste de l'acide aspartique.
- les composés se présentant sous la forme du composé de formule (V) : 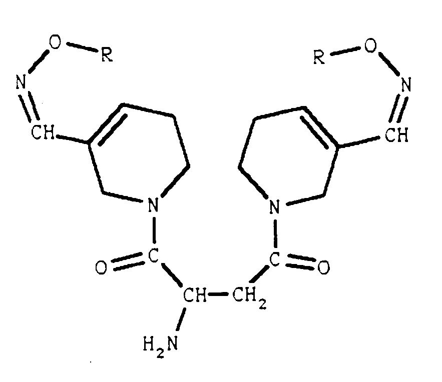 dans laquelle R conserve sa signification précédente, ainsi que leurs sels d'addition avec les acides organiques ou minéraux.

L'invention a tout spécialement pour objet le composé de formule (I) dont la préparation est donnée ci-après dans la partie expérimentale.

Les produits de l'invention présentent de très intéressantes propriétés pharmacologiques, notamment une activité cholinomimétique par voie orale et de longue durée d'action.

Les produits présentent de plus une bonne dissociation entre l'activité centrale et l'activité périphérique.

Il est bien connu que les troubles d'apprentissage et de la mémoire chez les personnes âgées sont surtout reliés à un déficit du système cholinergique central, en particulier dans la démence sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur des patients ayant un déficit de la mémoire (Sitaram N. et al. Science 201, 274, 1978) (Christie J.E. et al. Brit. J. Psychiatry 138, 46, 1981).

Une limitation à l'emploi thérapeutique de l'arécoline est liée au fait que ce produit a une très faible activité par voie orale et une courte durée d'action.

Les produits, objet de l'invention, ont montré, après administration par voie orale, une activité cholinomimétique centrale supérieure à celle de l'arécoline et avec une plus longue durée d'action.

L'invention a donc pour objet les produits de l'invention en tant que médicaments utiles notamment dans le traitement de la maladie d'Alzheimer ou de la démence sénile et également le traitement des troubles de la mémoire.

La posologie usuelle est variable selon l'affection en cause, le sujet traité et la voie d'administration ; elle peut être comprise entre 10 mg et 300 mg/jour, par exemple, entre 15 et 150 mg/jour en une ou plusieurs prises pour le produit de l'exemple 1 administré par voie orale.

La présente invention a également pour objet les compositions pharmaceutiques renfermant comme principe actif au moins un produit de formule (I) ou (V).

Les compositions pharmaceutiques selon l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet un acide de formule (II) : 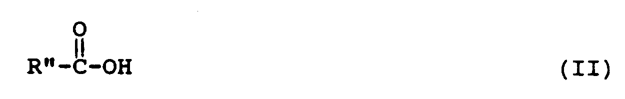
dans lequel R'' représente le reste de l'acide aminé ou du peptide R'CO₂H dans lequel le groupement amine terminal est bloqué et la fonction carboxylique a été activée,
avec le composé de formule (III) : 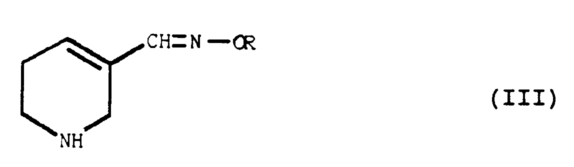
dans lequel R conserve sa signification précédente pour obtenir le composé de formule (I') : 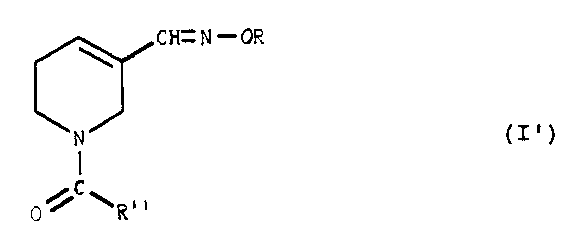
puis soumet le composé de formule (I') à l'action d'un agent de libération de la fonction amine pour obtenir le composé de formule (I) : 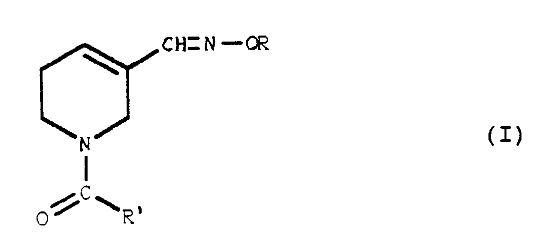
que l'on soumet si désiré, à l'action d'un acide pour en former le sel.

Dans un mode de réalisation préféré du procédé de l'invention :
A - la ou les fonctions amine sont protégées par des groupements facilement labiles utilisés couramment en chimie pharmaceutique par exemple les groupements : 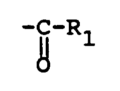 (conduisant aux dérivés N-acylés) 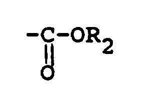 (conduisant aux carbamates)
   CH₂NHCOR₃ (conduisant aux bases de Mannich)
   -CHR₅-O-CO-R₄ (conduisant aux dérivés N-acyloxyalkylés)
   -CR₇=CHCO₂R₆ (conduisant aux énaminones)
   les radicaux R₁, R₂, R₃, R₄, R₅, R₆ et R₇ représentant des radicaux hydrocarbonés.
   Comme groupement préféré pour bloquer la fonction amine, on peut citer particulièrement le 9-fluorényl méthyloxy carbonyl (FMOC). De nombreux FMOC L acides aminés sont d'ailleurs disponibles dans le commerce par exemple la FMOC-L-alanine.
   On peut aussi utiliser comme groupement protecteur préféré le groupement :
   a) benzyloxycarbonyl 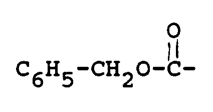
   b) p-méthoxybenzyloxycarbonyl 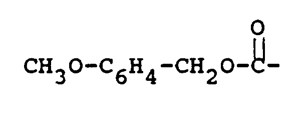
   c) t-butyloxycarbonyl 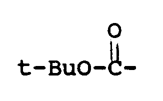
   d) diphénylisopropyloxycarbonyl 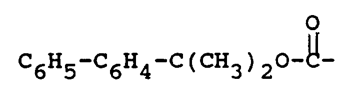
   e) triphénylméthyl (C₆H₅)₃C-
   f) o-nitrobenzènesulfényl NO₂C₆H₄S-
   g) triméthylsilyléthyloxycarbonyl 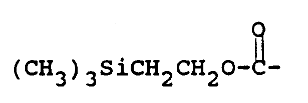
   h) di-p-nitrophényléthyloxycarbonyl 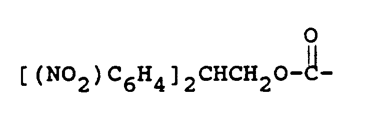
B - Pour activer la fonction carboxyle, on peut transformer l'acide en halogénure d'acide par exemple en chlorure d'acide en utilisant le chlorure de thionyle au sein d'un solvant inerte comme le chlorure de méthylène ou le chloroforme. On peut également transformer l'acide aminé en d'autres dérivés par exemple :
   - en azidure 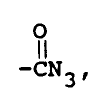
   - en anhydride mixte,
   - en esters, par exemple en ester de p-nitro phényle, en esters de polyhalogéno phényle, en esters de 1-hydroxy benzotriazole, ou encore de N-hydroxy succinimide, de 2-hydroxy pyridine ou de 2-mercapto pyridine.
C - La réaction du composé de formule (II) avec le composé de formule (III) a lieu au sein d'un solvant inerte comme le chlorure de méthylène, le chloroforme, le dichloro éthane, le benzène, le toluène, éventuellement en présence d'une base comme TEA (triéthylamine), DABCO (triéthylène diamine), DBN (1,5-diazabicyclo [4,3,0] non-5-ène), DBU (1,8-diazabicyclo [5,4,0] undec-7-ène).
D - La réaction de libération de la fonction amine protégée par le groupement FMOC, a lieu en utilisant une amine secondaire comme la diéthylamine, la pipéridine ou la morpholine, et en opérant à la température ambiante.
   On peut également libérer la fonction amine protégée par les autres groupements au moyen d'une hydrogénolyse, d'une acidolyse ou en utilisant le fluorure de tétrabutylammonium Bu₄NF.
E - La formation de sels est réalisée par addition d'acides de façon classique.

Les composés de formule (III) utilisés comme produits de départ sont des produits connus, décrits et revendiqués dans la demande de brevet européen 239.445.

L'invention a plus particulièrement pour objet un procédé, caractérisé en ce que l'on soumet un composé de formule (II_{A}) : 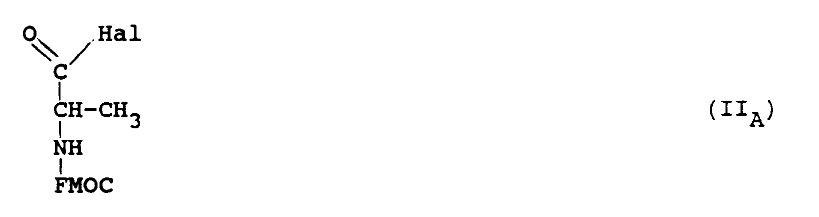
dans lequel Hal représente un atome d'halogène et FMOC représente le radical fluorénylméthoxycarbonyle, à l'action d'un composé de formule (III) : 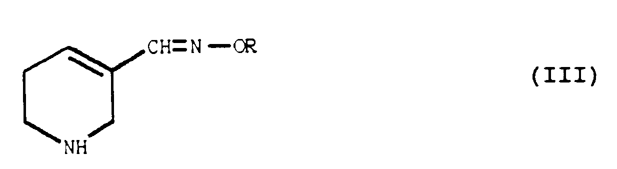
pour obtenir le composé de formule (IV) : 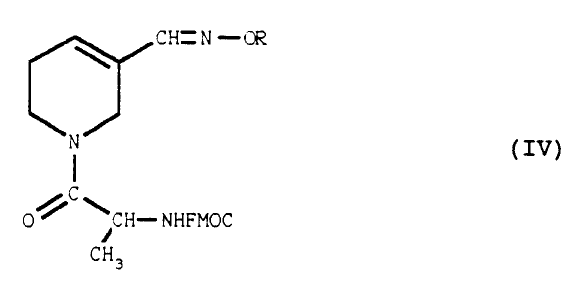
dont on libère la fonction amine pour obtenir le composé de formule (I_{A}) correspondant : 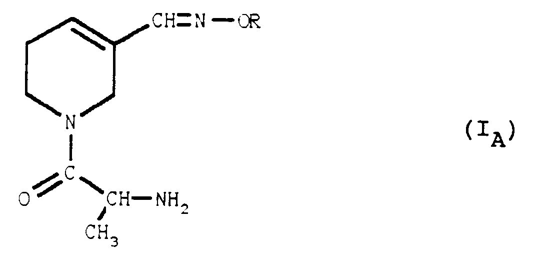
Dans un mode de réalisation préféré :
- Hal est un atome de chlore,
- la réaction de condensation du composé de formule (II_{A}) et de formule (III) est réalisée au sein du dichloroéthane en présence d'une base comme le DBU, le DBN ou la TEA.
- la réaction de libération de la fonction amine a lieu par action d'une base comme la morpholine, à la température ambiante.

L'exemple suivant illustre l'invention.

### EXEMPLE 1 : (L)-2-amino 1-[3-(méthoxyimino) méthyl 1,2,5,6-tétrahydro 1-pyridinyl] 1-propanone

### STADE A : N-[2-[3-(méthoxyimino) méthyl 1,2,5,6-tétrahydro 1-pyridinyl] 1-méthyl 2-oxoéthyl]-carbamate de 9-fluorénylméthyle.

On dissout 4,6 g de chlorure de 2-(9-fluorényl méthoxycarbonylamino) L-propanoyl dans 50 cm³ de dichlorométhane, ajoute lentement une solution comprenant 1,78 g de 1,2,5,6-tétrahydro-3-pyridine carboxaldéhyde O-méthyloxime et 1,99 g de 1,8-diazabicyclo [5,4,0] undec-7-ène dans 40 cm³ de dichlorométhane. On agite 1 heure à température ambiante, évapore sous pression réduite, reprend avec de l'eau et extrait à l'acétate d'éthyle. On sèche et évapore les solvants. Après chromatographie sur silice (éluant : acétate d'éthyle-hexane 1-1), on obtient 3,92 g d'une huile qui se transforme en solide, sous pression réduite.

### STADE B : (L-) 2-amino 1-[3-(méthoxyimino) méthyl 1,2,5,6-tétrahydro 1-pyridinyl] 1-propanone.

On agite pendant 40 minutes à température ambiante, 3,8 g de N-[2-[3-(méthoxyimino) méthyl 1,2,5,6-tétrahydro-1-pyridinyl] 1-méthyl 2-oxoéthyl] carbamate de 9-fluorénylméthyle préparé au stade A et 50 cm³ de morpholine. On élimine sous pression réduite la majeure partie de la morpholine, verse dans 350 cm³ d'eau froide et élimine par filtration la N-9-fluorénylméthylmorpholine. On concentre le filtrat jusqu'à un volume d'environ 200 cm³, extrait plusieurs fois au dichlorométhane, sèche et évapore le solvant. On abandonne sous pression réduite pendant 24 heures, chromatographie sur silice (éluant : chloroforme-méthanol 7-3) et obtient 1,8 g de produit huileux que l'on salifie par addition de 4,3 cm³ d'éthanol chlorhydrique 2N. Après cristallisation dans l'éthanol et dans l'éther, on obtient 1,87 g de produit attendu.
F = 215-217°C (décomp.).

| Analyse : C₁₀H₁₇N₃O₂,HCl : 247,726 | | | | | | |
|---|---|---|---|---|---|---|
| Calculé | C% | 48,49 | H% | 7,32 | N% | 16,96 |
| Trouvé | | 48,51 | | 7,36 | | 16,99 |

### Préparation du chlorure de 2-(9-fluorényl méthoxy carbonylamino) L-propanoyle utilisé au départ de l'exemple.

On chauffe au reflux pendant 20 minutes un mélange constitué de 5 g de 9-fluorénylmethoxycarbonyl-(L)-alanine et de 9,5 cm³ de chlorure de thionyle dans 60 cm³ de dichlorométhane. On élimine le solvant, reprend le résidu dans un peu de dichlorométhane, précipite au moyen d'hexane et obtient 4,83 g de produit attendu. F = 108-110°C.

### ETUDE PHARMACOLOGIQUE

### Toxicité aigüe.

L'essai est réalisé sur des souris mâles (CD₁ Charles Rivers) de 22 à 24 g, à jeun depuis 16 heures. On administre les produits par voie orale à la dose de 1000, 500, 250 et 125 mg/kg. On note la mortalité pendant les 7 jours suivant le traitement.

### Test de l'iléon isolé de cobaye

On prélève des fragments d'iléon de cobayes tués par décapitation. L'iléon isolé est placé dans 10 cm³ de solution de Tyrode à 37°C et aéré par un mélange d'oxygène (95%) et de gaz carbonique (5%). Les contractions dues aux produits sont enregistrées à l'aide d'un capteur relié à un polygraphe. Les produits à tester sont ajoutés, aux concentrations comprises entre 1.10⁻³M et 1.10⁻⁸M/l.

Les produits présentant un effet contracturant sont testés vis à vis de l'atropine et de l'hexaméthonium pour établir si l'activité est de type "muscarinique" ou "nicotinique".

L'activité antagoniste éventuelle des produits est testée vis à vis de l'acétylcholine.

L'activité agoniste est exprimée en pD₂ (logarithme négatif de la dose qui produit 50% de l'effet maximum).

L'activité antagoniste est exprimée en DE₅₀ (dose réduisant de 50% la réponse maximale induite par l'acétylcholine).

### Activité diarrhéique.

Le test est réalisé sur des souris mâles (CD₁ Charles Rivers) pesant 25 à 30 g, à jeun depuis 6 heures. Le produit dissous à 5% dans du Méthocel® est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes :
- 0 :: consistance ferme,
- 1 :: fèces légèrement molles avec ou sans auréole humide,
- 2 :: fèces légèrement molles avec présence d'un cercle humide bien défini,
- 3 :: fèces molles avec présence d'un grand cercle humide,
- 4 :: fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57, 261, 1944).

### Activité hypothermique.

Le test est réalisé sur des souris mâles (CD₁ Charles Rivers) pesant 25 à 30 g, à jeun depuis 6 heures.

La température du corps est notée au moyen d'un thermocouple placé dans le rectum à environ 1,5 cm et relié à un enregistreur de température électrique.

Les produits sont administrés par voie orale ou sous-cutanée et les températures sont notées à l'instant 0 et 30 minutes, 1 heure, 2 heures et 3 heures après traitement.

On évalue le degré d'hypothermie comme la différence entre les animaux traités et les témoins et on détermine la dose nécessaire pour réduire de 1°C la température du corps.

On détermine la durée d'action des produits en utilisant des doses capables de réduire la température de 1°C à 1,5°C.

Les résultats obtenus lors des essais biologiques ont montrés qu'administé par voie orale, le produit de l'exemple 1 présente une activité cholinomimétique importante et de longue durée d'action, le produit de l'exemple 1 présente de plus une importante dissociation entre l'activité centrale et l'activité périphérique.

## Revendications (Revendications pour l'(les) Etat(s) contractant(s) suivant(s): AT, BE, CH, DE, DK, FR, GB, IT, LI, LU, NL, SE)

1. Les composés de formule (I) : 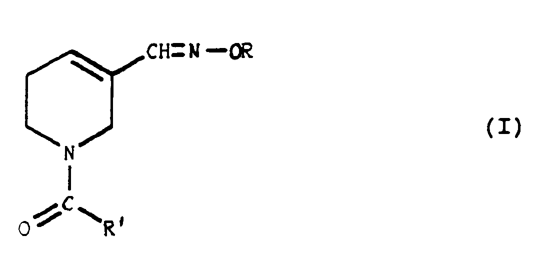 dans laquelle :
- R représente un atome d'hydrogène, un radical alkyle linéaire, ramifié ou cyclique, saturé ou insaturé renfermant jusqu'à 8 atomes de carbone,
- R' représente le reste d'un acide aminé ou d'un peptide à 2, 3 ou 4 acides aminés,
- le groupement CO provient du groupement carboxyle alpha de l'acide aminé ou du groupement CO terminal du peptide,
ainsi que leurs sels d'addition avec les acides organiques ou minéraux.

2. Les composés de formule (I) tels que définis à la revendication 1, dans lesquels R représente un radical alkyle linéaire, saturé ou insaturé renfermant jusqu'à 4 atomes de carbone, ainsi que leurs sels d'addition avec les acides organiques ou minéraux.

3. Les composés de formule (I) tels que définis à la revendication 2, dans lesquels R représente un radical méthyle ainsi que leurs sels d'addition avec les acides organiques ou minéraux.

4. Les composés de formule (I) tels que définis à l'une quelconque des revendications 1 à 3, dans lesquels R' représente le reste d'un acide alpha-amino carboxylique de configuration L ainsi que leurs sels d'addition avec les acides organiques ou minéraux.

5. Les composés de formule (I) tels que définis à l'une quelconque des revendications 1 à 4 dans lesquels R' représente le reste de l'alanine ainsi que leurs sels d'addition avec les acides organiques ou minéraux.

6. Le composé défini à la revendication 5 dont le nom suit :
- (L-) 2-amino 1-[3-(méthoxyimino) méthyl 1,2,5,6-tétrahydro 1-pyridinyl] 1-propanone.

7. Les composés de formule (I) tels que définis à l'une quelconque des revendications 1 à 4, dans lesquels R' représente le reste d'un acide aminé renfermant deux fonctions acides, ainsi que leurs sels d'addition avec les acides organiques ou minéraux.

8. Les composés de formule (I) tels que définis à la revendication 7, dans lesquels R' représente le reste de l'acide aspartique, ainsi que leurs sels d'addition avec les acides organiques ou minéraux.

9. Les composés de formule (V) se présentant sous la forme du composé de formule : 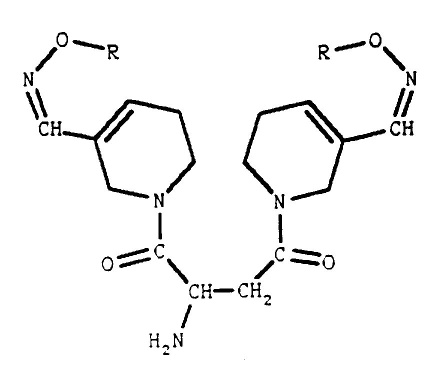 dans laquelle R est tel que défini dans la revendication 1.

10. A titre de médicaments, les composés de formule (I) ou (V) tels que définis à l'une quelconque des revendications 1 à 5 et 7 à 9, ainsi que leurs sels avec les acides organiques ou minéraux, pharmaceutiquement acceptables.

11. A titre de médicament, le composé de formule (I) défini à la revendication 6, ainsi que ses sels d'addition avec les acides organiques ou minéraux.

12. Les compositions pharmaceutiques renfermant comme principe actif au moins un médicament défini à la revendication 10 ou 11.

13. Procédé de préparation des composés de formule (I) tels que définis à la revendication 1, caractérisé en ce que l'on soumet un acide de formule (II) : 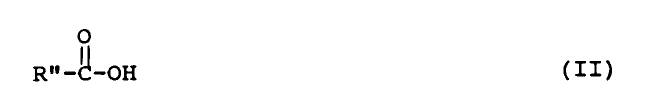 dans lequel R'' représente le reste de l'acide aminé ou du peptide R'CO₂H dans lequel le groupement amine terminal est bloqué et la fonction carboxylique a été activée,
avec le composé de formule (III) : 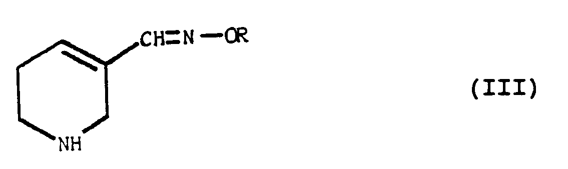 dans lequel R conserve sa signification précédente pour obtenir le composé de formule (I') : 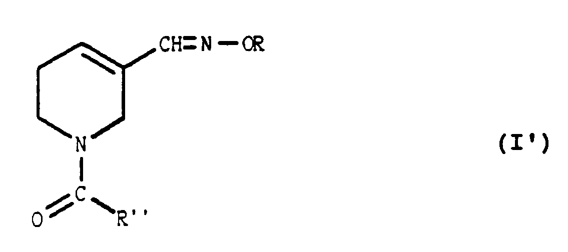 puis soumet le composé de formule (I') à l'action d'un agent de libération de la fonction amine pour obtenir le composé de formule (I) : 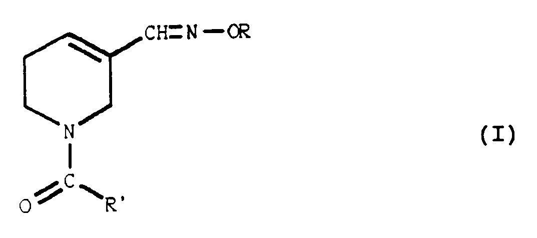 que l'on soumet si désiré, à l'action d'un acide pour en former le sel.

14. Procédé selon la revendication 13 pour la préparation des composés de formule (I) tels que définis à la revendication 5, caractérisé en ce que l'on soumet un composé de formule (II_{A}) : 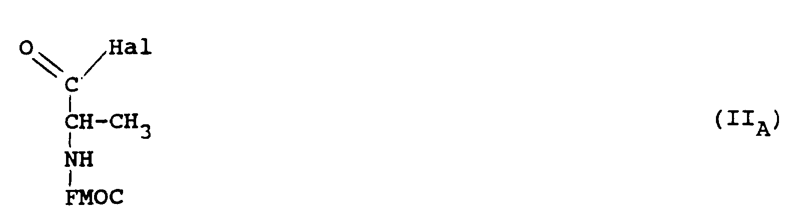 dans lequel Hal représenté un atome d'halogène et FMOC représente le radical fluorénylméthoxycarbonyle, à l'action d'un composé de formule (III) : 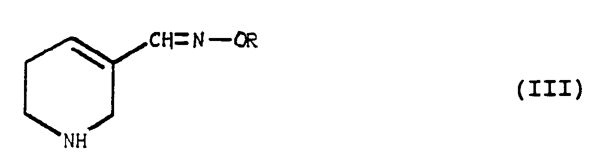 pour obtenir le composé de formule (IV) : 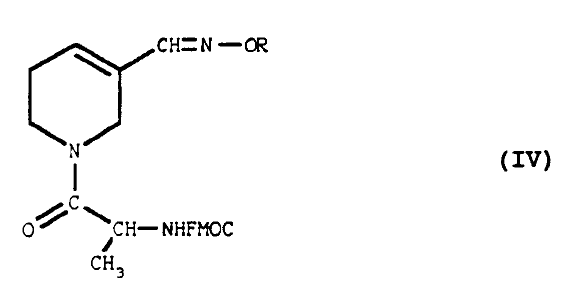 dont on libère la fonction amine pour obtenir le composé de formule (I_{A}) correspondant :

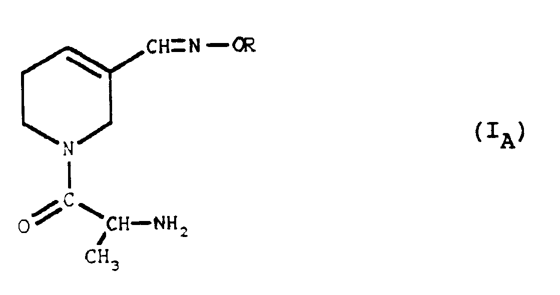

## Revendications (Revendications pour l'(les) Etat(s) contractant(s) suivant(s): ES)

1. Procédé pour préparer les composés de formule (I) : 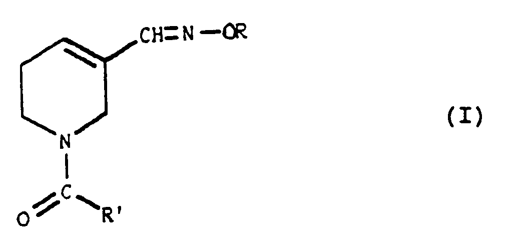 dans laquelle :
- R représente un atome d'hydrogène, un radical alkyle linéaire, ramifié ou cyclique, saturé ou insaturé renfermant jusqu'à 8 atomes de carbone,
- R' représente le reste d'un acide aminé ou d'un peptide à 2, 3 ou 4 acides aminés,
- le groupement CO provient du groupement carboxyle alpha de l'acide aminé ou du groupement CO terminal du peptide,
ainsi que leurs sels d'addition avec les acides organiques ou minéraux, caractérisé en ce que l'on soumet un acide de formule (II) : 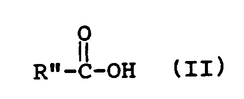 dans lequel R'' représente le reste de l'acide aminé ou du peptide R'CO₂H dans lequel le groupement amine terminal est bloqué et la fonction carboxylique a été activée, avec le composé de formule (III) : 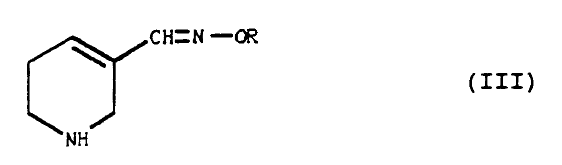 dans lequel R conserve sa signification précédente pour obtenir le composé de formule (I') : 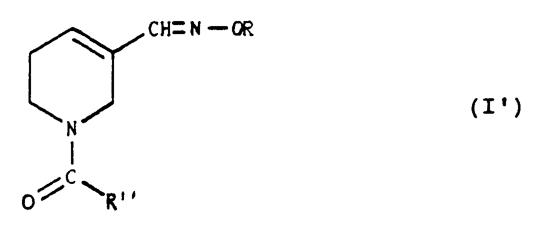 puis soumet le composé de formule (I') à l'action d'un agent de libération de la fonction amine pour obtenir le composé de formule (I) : 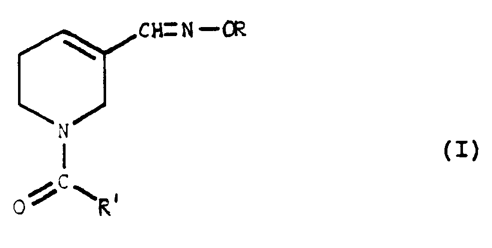 que l'on soumet si désiré, à l'action d'un acide pour en former le sel.

2. Procédé selon la revendication 1, caractérisé en ce que l'on utilise au départ un produit de formule (III) dans laquelle R représente un radical alkyle linéaire, saturé ou insaturé renfermant jusqu'à 4 atomes de carbone.

3. Procédé selon la revendication 1, caractérisé en ce que l'on utilise au départ un produit de formule (III) dans laquelle R représente un radical méthyle.

4. Procédé selon la revendication 1, caractérisé en ce que l'on utilise au départ un produit de formule (II) dans laquelle R'' représente le reste d'un acide alpha-amino carboxylique de configuration L.

5. Procédé selon la revendication 1, caractérisé en ce que l'on soumet un composé de formule (II_{A}) : 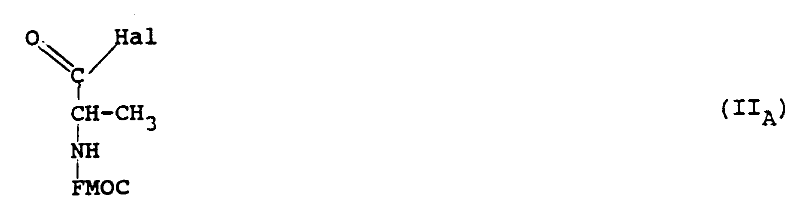 dans lequel Hal représente un atome d'halogène et FMOC représente le radical fluorénylméthoxycarbonyle, à l'action d'un composé de formule (III) : 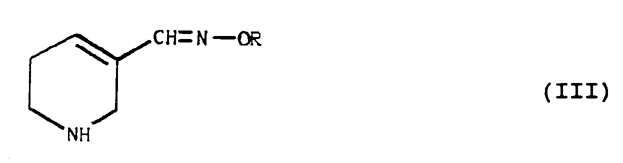 pour obtenir le composé de formule (IV) : 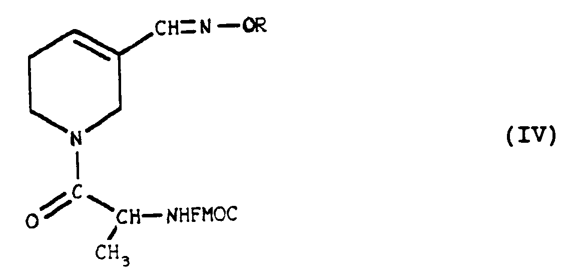 dont on libère la fonction amine pour obtenir le composé de formule (I_{A}) correspondant :

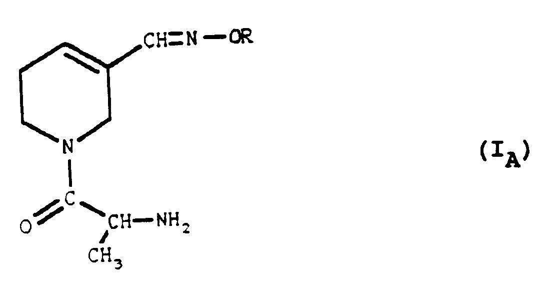

6. Procédé selon la revendication 5, caractérisé en ce que l'on met en oeuvre des produits de formule (II_{A}) et (III) choisis de manière telle que l'on prépare le (L-) 2-amino 1-[3-(méthoxyimino) méthyl 1,2,5,6-tétrahydro 1-pyridinyl] 1-propanone.

7. Procédé selon la revendication 1, caractérisé en ce que l'on utilise au départ un produit de formule (II) dans laquelle R'' représente le reste d'un acide aminé renfermant deux fonctions acides.

8. Procédé selon la revendication 7, caractérisé en ce que le produit de formule (II) que l'on utilise au départ est l'acide aspartique.

9. Procédé caractérisé en ce que l'on met en oeuvre un produit de formule (II') et (III) choisi de manière telle que l'on prépare un produit se présentant sous la forme du composé de formule (V): 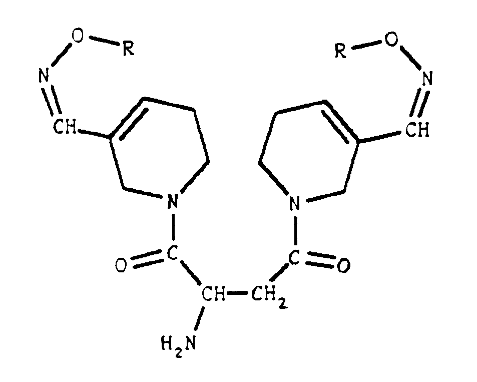 dans laquelle R est tel que défini dans la revendication 1.

## Revendications (Revendications pour l'(les) Etat(s) contractant(s) suivant(s): GR)

1. Procédé pour préparer les composés de formule (I) : 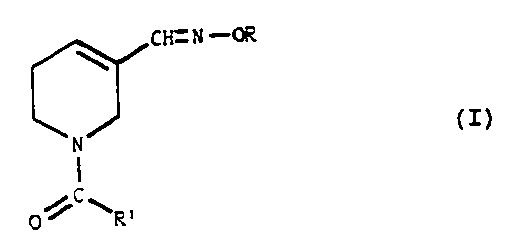 dans laquelle :
- R représente un atome d'hydrogène, un radical alkyle linéaire, ramifié ou cyclique, saturé ou insaturé renfermant jusqu'à 8 atomes de carbone,
- R' représente le reste d'un acide aminé ou d'un peptide à 2, 3 ou 4 acides aminés,
- le groupement CO provient du groupement carboxyle alpha de l'acide aminé ou du groupement CO terminal du peptide,
ainsi que leurs sels d'addition avec les acides organiques ou minéraux, caractérisé en ce que l'on soumet un acide de formule (II) : 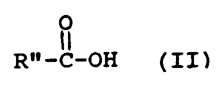 dans lequel R'' représente le reste de l'acide aminé ou du peptide R'CO₂H dans lequel le groupement amine terminal est bloqué et la fonction carboxylique a été activée,
avec le composé de formule (III) : 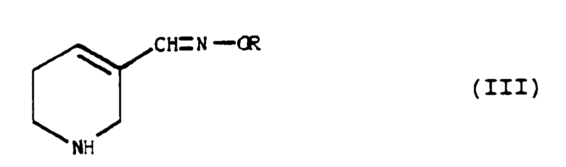 dans lequel R conserve sa signification précédente pour obtenir le composé de formule (I') : 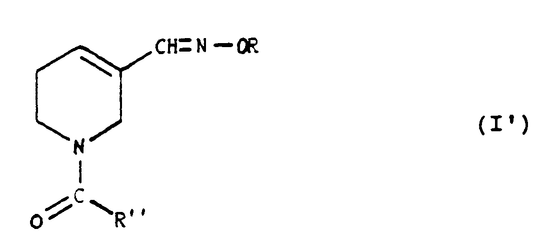 puis soumet le composé de formule (I') à l'action d'un agent de libération de la fonction amine pour obtenir le composé de formule (I) : 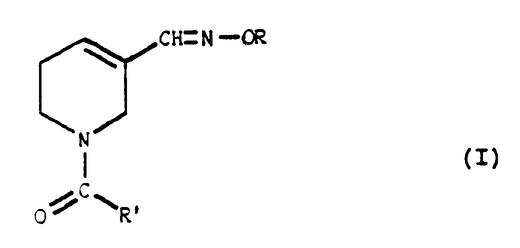 que l'on soumet si désiré, à l'action d'un acide pour en former le sel.

2. Procédé selon la revendication 1, caractérisé en ce que l'on utilise au départ un produit de formule (III) dans laquelle R représente un radical alkyle linéaire, saturé ou insaturé renfermant jusqu'à 4 atomes de carbone.

3. Procédé selon la revendication 1, caractérisé en ce que l'on utilise au départ un produit de formule (III) dans laquelle R représente un radical méthyle.

4. Procédé selon la revendication 1, caractérisé en ce que l'on utilise au départ un produit de formule (II) dans laquelle R'' représente le reste d'un acide alpha-amino carboxylique de configuration L.

5. Procédé selon la revendication 1, caractérisé en ce que l'on soumet un composé de formule (II_{A}) : 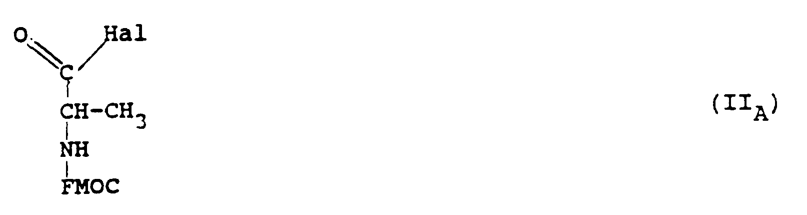 dans lequel Hal représente un atome d'halogène et FMOC représente le radical fluorénylméthoxycarbonyle, à l'action d'un composé de formule (III) : 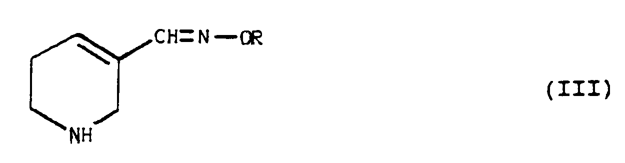 pour obtenir le conposé de formule (IV) : 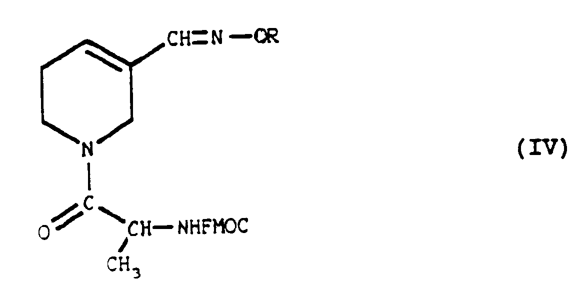 dont on libère la fonction amine pour obtenir le composé de formule (I_{A}) correspondant :

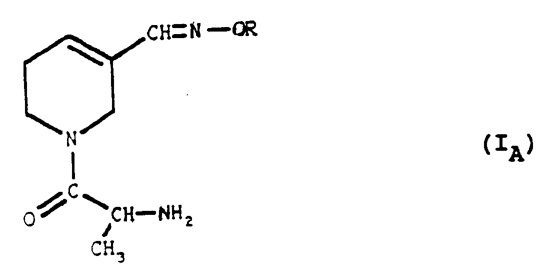

6. Procédé selon la revendication 5, caractérisé en ce que l'on met en oeuvre des produits de formule (II_{A}) et (III) choisis de manière telle que l'on prépare le (L-) 2-amino 1-[3-(méthoxyimino) méthyl 1,2,5,6-tétrahydro 1-pyridinyl] 1-propanone.

7. Procédé selon la revendication 1, caractérisé en ce que l'on utilise au départ un produit de formule (II) dans laquelle R'' représente le reste d'un acide aminé renfermant deux fonctions acides.

8. Procédé selon la revendication 7, caractérisé en ce que le produit de formule (II) que l'on utilise au départ est l'acide aspartique.

9. Procédé caractérisé en ce que l'on met en oeuvre un produit de formule (II') et (III) choisi de manière telle que l'on prépare un produit se présentant sous la forme du composé de formule (V) : 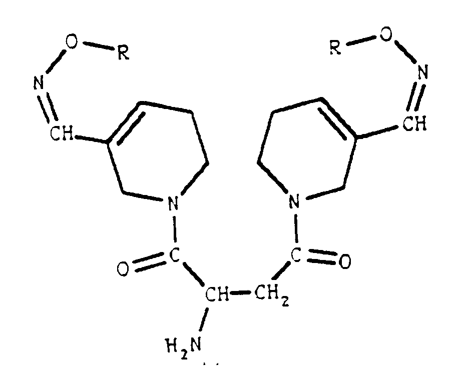 dans laquelle R est tel que défini dans la revendication 1.

10. Procédé de préparation de compositions pharmaceutiques caractérisé en ce que l'on met à titre de principe actif l'un ) au moins de produits de formule (I) ou (V) tels que définis à l'une quelconque des revendications 1 à 5 et 7 à 9 ou l'un au moins de leurs sels d'addition avec les acides organiques ou minéraux pharmaceutiquement acceptables sous une forme destinée à cet usage.

11. Procédé de préparation de compositions pharmaceutiques caractérisé en ce que l'on met à titre de principe actif le produit de formule (I) tel que défini à la revendication 6 sous une forme destinée à cet usage.

## Claims (Claims for the following Contracting State(s): AT, BE, CH, DE, DK, FR, GB, IT, LI, LU, NL, SE)

1. The compounds of formula (I): 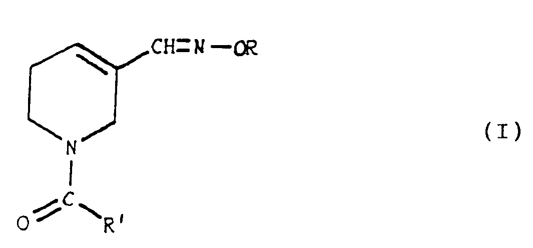 in which:
- R represents a hydrogen atom, a saturated or unsaturated, linear, branched or cyclic alkyl radical containing up to 8 carbon atoms,
- R' represents the remainder of an amino acid or of a peptide with 2, 3 or 4 amino acids,
- the CO group comes from the alpha carboxyl group of the amino acid or from the terminal CO group of the peptide, as well as their addition salts with organic or mineral acids.

2. The compounds of formula (I) as defined in claim 1, in which R represents a saturated or unsaturated, linear alkyl radical containing up to 4 carbon atoms, as well as their addition salts with organic or mineral acids.

3. The compounds of formula (I) as defined in claim 2, in which R represents a methyl radical as well as their addition salts with organic or mineral acids.

4. The compounds of formula (I) as defined in any one of claims 1 to 3, in which R' represents the remainder of a carboxylic alpha-amino acid of L configuration as well as their addition salts with organic or mineral acids.

5. The compounds of formula (I) as defined in any one of claims 1 to 4 in which R' represents the remainder of alanine as well as their addition salts with organic or mineral acids.

6. The compound defined in claim 5 whose name follows:
- (L-) 2-amino 1-[3-(methoxyimino) methyl 1,2,5,6-tetrahydro 1-pyridinyl] 1-propanone.

7. The compounds of formula (I) as defined in any one of claims 1 to 4, in which R' represents the remainder of an amino acid containing two acid functions, as well as their addition salts with organic or mineral acids.

8. The compounds of formula (I) as defined in claim 7, in which R' represents the remainder of aspartic acid, as well as their addition salts with organic or mineral acids.

9. The compounds of formula (V) which are presented in the form of the compound of formula: 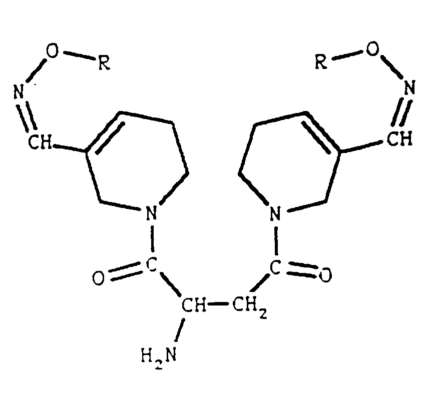 in which R is as defined in claim 1.

10. As medicaments, the compounds of formula (I) or (V) as defined in any one of claims 1 to 5 and 7 to 9, as well as their salts with pharmaceutically acceptable organic or mineral acids.

11. As a medicament, the compound of formula (I) defined in claim 6, as well as its addition salts with organic or mineral acids.

12. Pharmaceutical compositions containing as active ingredient at least one medicament defined in claim 10 or 11.

13. Preparation process for the compounds of formula (I) as defined in claim 1, characterized in that an acid of formula (II): 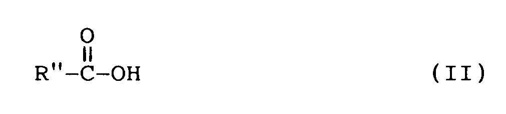 in which R'' represents the remainder of the amino acid or the peptide R'CO₂H in which the terminal amine group is blocked and the carboxylic function has been activated, is reacted with the compound of formula (III): 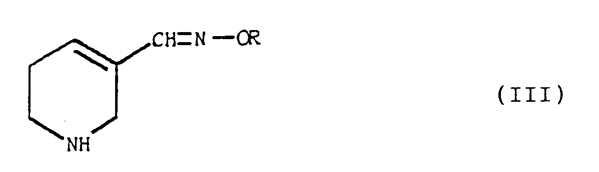 in which R retains its previous meaning, in order to obtain the compound of formula (I'): 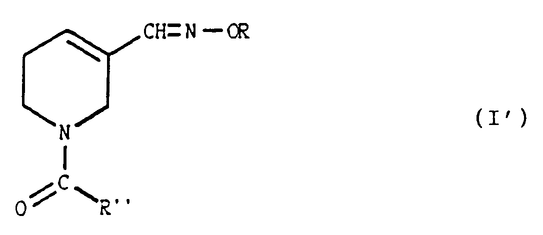 then the compound of formula (I') is subjected to the action of a releasing agent of the amine function in order to obtain the compound of formula (I): 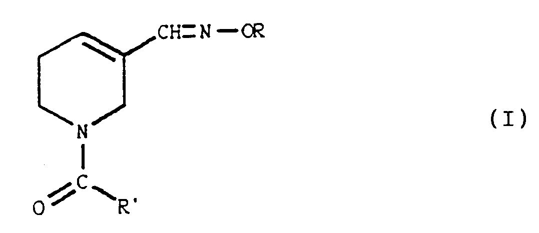 which is subjected, if desired, to the action of an acid in order to form the salt.

14. Process according to claim 13 for the preparation of compounds of formula (I) as defined in claim 5, characterized in that a compound of formula (II_{A}): 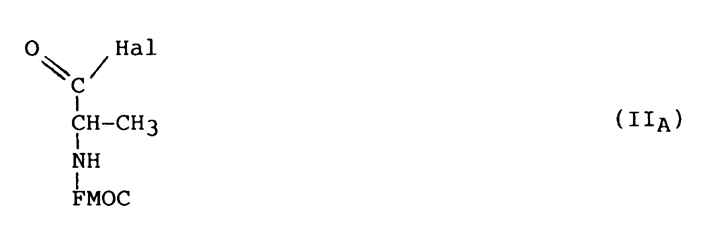 in which Hal represents a halogen atom and FMOC represents the fluorenylmethoxycarbonyl radical, is subjected to the action of a compound of formula (III): 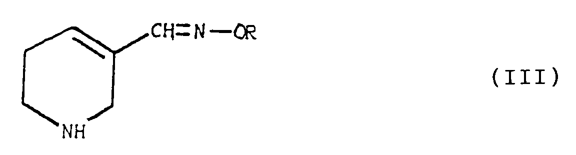 in order to obtain the compound of formula (IV): 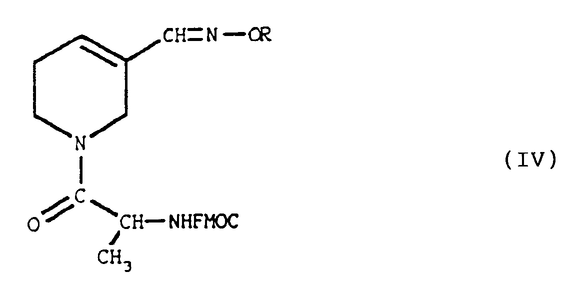 the amine function of which is released in order to obtain the corresponding compound of formula (I_{A}):

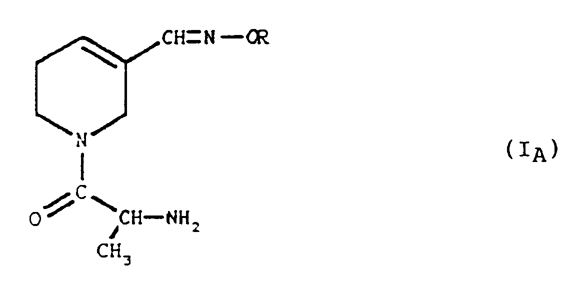

## Claims (Claims for the following Contracting State(s): ES)

1. Process for preparing the compounds of formula (I): 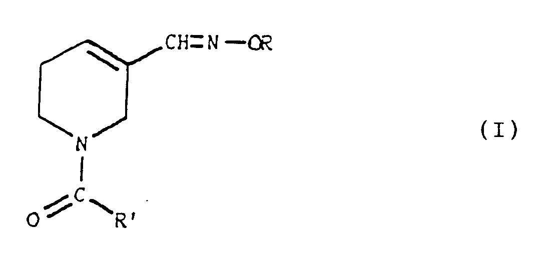 in which:
- R represents a hydrogen atom, a saturated or unsaturated, linear, branched or cyclic alkyl radical containing up to 8 carbon atoms,
- R' represents the remainder of an amino acid or of a peptide with 2, 3 or 4 amino acids,
- the CO group comes from the alpha carboxyl group of the amino acid or from the terminal CO group of the peptide,
as well as their addition salts with organic or mineral acids, characterized in that an acid of formula (II): 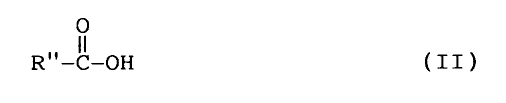 in which R'' represents the remainder of the amino acid or the peptide R'CO₂H in which the terminal amine group is blocked and the carboxylic function has been activated, is reacted with the compound of formula (III): 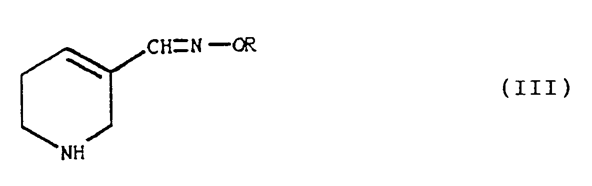 in which R retains its previous meaning, in order to obtain the compound of formula (I'): 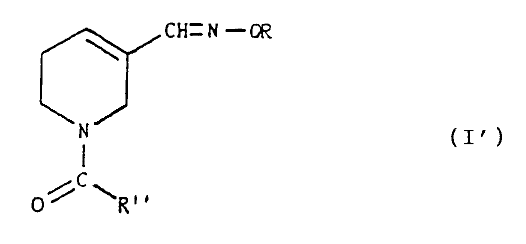 then the compound of formula (I') is subjected to the action of a releasing agent of the amine function in order to obtain the compound of formula (I): 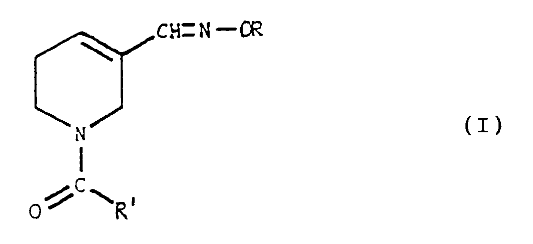 which is subjected, if desired, to the action of an acid in order to form the salt.

2. Process according to claim 1, characterized in that a product of formula (III) is used at the start in which R represents a saturated or unsaturated linear alkyl radical containing up to 4 carbon atoms.

3. Process according to claim 1, characterized in that a product of formula (III) is used at the start in which R represents a methyl radical.

4. Process according to claim 1, characterized in that a product of formula (II) is used at the start in which R'' represents the remainder of a carboxylic alpha-amino acid of L configuration.

5. Process according to claim 1, characterized in that a compound of formula (II_{A}): 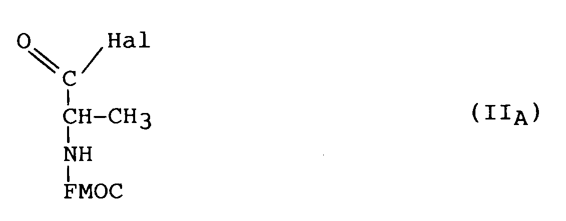 in which Hal represents a halogen atom and FMOC represents the fluorenylmethoxycarbonyl radical, is subjected to the action of a compound of formula (III): 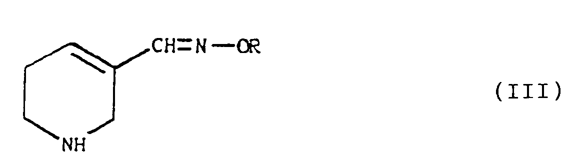 in order to obtain the compound of formula (IV): 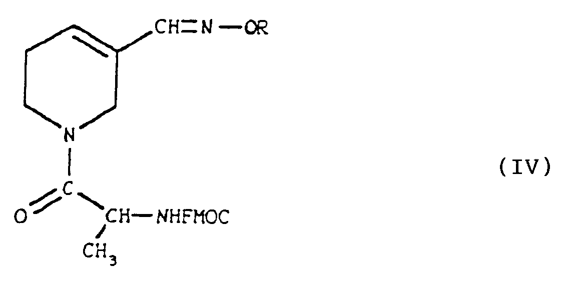 the amine function of which is released in order to obtain the corresponding compound of formula (I_{A}):

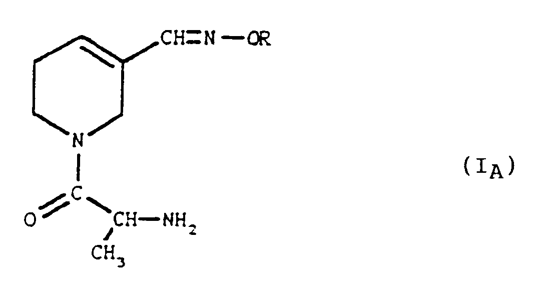

6. Process according to claim 5, characterized in that the products of formulae (II_{A}) and (III) are used, chosen in such a manner that (L-) 2-amino 1-[3-(methoxyimino) methyl 1,2,5,6-tetrahydro 1-pyridinyl] 1-propanone is prepared.

7. Process according to the claim 1, characterized in that a product of formula (II) is used at the start in which R'' represents the remainder of an amino acid containing two acid functions.

8. Process according to claim 7, characterized in that the product of formula (II) which is used at the start is aspartic acid.

9. Process characterized in that a product of formulae (II') and (III) is used, chosen in such a manner that a product is prepared which is presented in the form of the compound of formula (V): 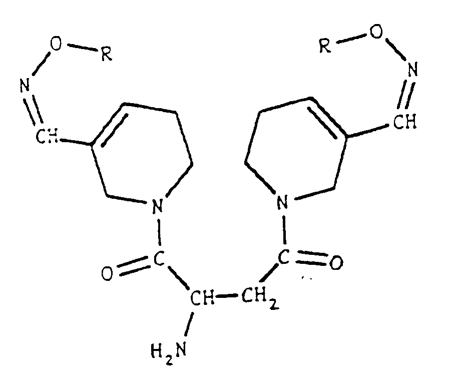 in which R is as defined in claim 1.

## Claims (Claims for the following Contracting State(s): GR)

1. Process for preparing the compounds of formula (I): 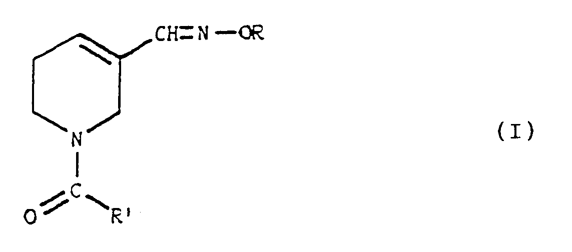 in which:
- R represents a hydrogen atom, a saturated or unsaturated, linear, branched or cyclic alkyl radical containing up to 8 carbon atoms,
- R' represents the remainder of an amino acid or of a peptide with 2, 3 or 4 amino acids,
- the CO group comes from the alpha carboxyl group of the amino acid or from the terminal CO group of the peptide,
as well as their addition salts with organic or mineral acids, characterized in that an acid of formula (II): 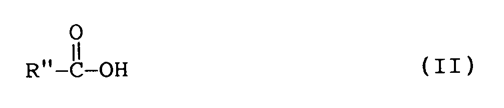 in which R'' represents the remainder of the amino acid or the peptide R'CO₂H in which the terminal amine group is blocked and the carboxylic function has been activated, is reacted with the compound of formula (III): 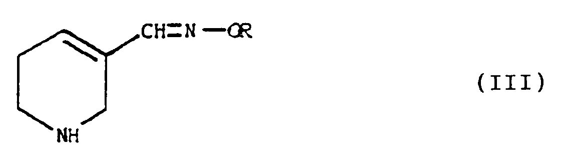 in which R retains its previous meaning, in order to obtain the compound of formula (I'): 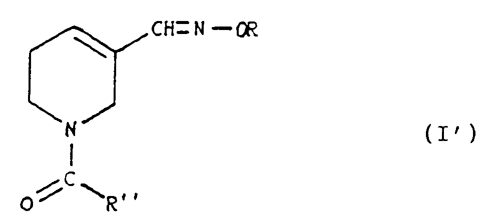 then the compound of formula (I') is subjected to the action of a releasing agent of the amine function in order to obtain the compound of formula (I): 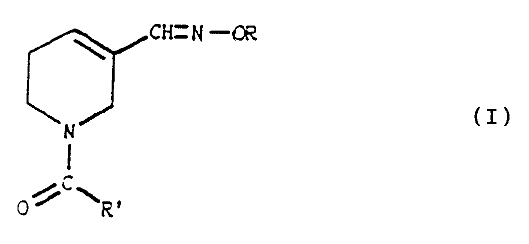 which is subjected, if desired, to the action of an acid in order to form the salt.

2. Process according to claim 1, characterized in that a product of formula (III) is used at the start in which R represents a saturated or unsaturated linear alkyl radical containing up to 4 carbon atoms.

3. Process according to claim 1, characterized in that a product of formula (III) is used at the start in which R represents a methyl radical.

4. Process according to claim 1, characterized in that a product of formula (II) is used at the start in which R'' represents the remainder of a carboxylic alpha-amino acid of L configuration.

5. Process according to claim 1, characterized in that a compound of formula (II_{A}): 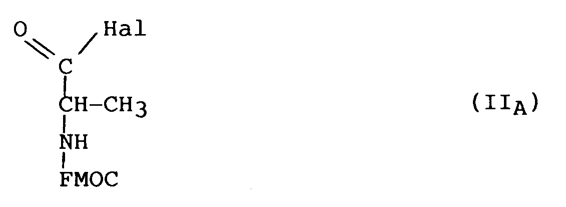 in which Hal represents a halogen atom and FMOC represents the fluorenylmethoxycarbonyl radical, is subjected to the action of a compound of formula (III): 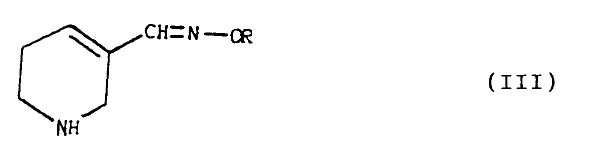 in order to obtain the compound of formula (IV): 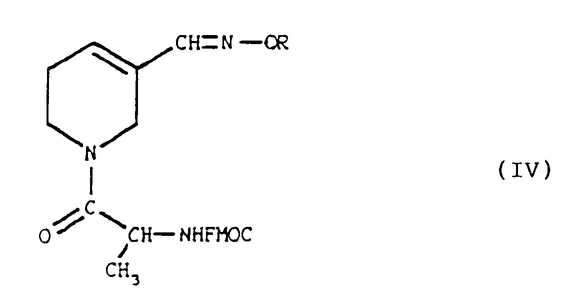 the amine function of which is released in order to obtain the corresponding compound of formula (I_{A}):

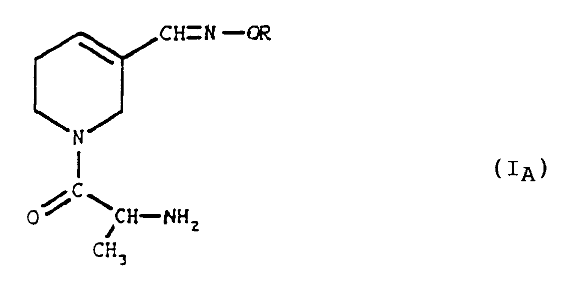

6. Process according to claim 5, characterized in that the products of formulae (II_{A}) and (III) are used, chosen in such a manner that (L-) 2-amino 1-[3-(methoxyimino) methyl 1,2,5,6-tetrahydro 1-pyridinyl] 1-propanone is prepared.

7. Process according to the claim 1, characterized in that a product of formula (II) is used at the start in which R'' represents the remainder of an amino acid containing two acid functions.

8. Process according to claim 7, characterized in that the product of formula (II) which is used at the start is aspartic acid.

9. Process characterized in that a product of formulae (II') and (III) is used, chosen in such a manner that a product is prepared which is presented in the form of the compound of formula (V): 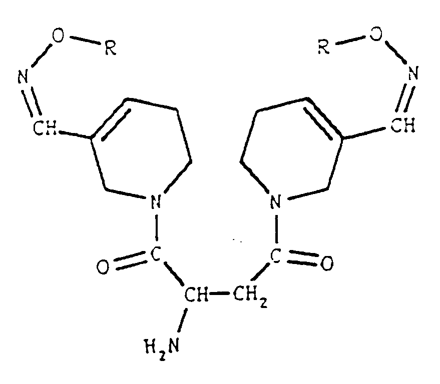 in which R is as defined in claim 1.

10. Preparation process for pharmaceutical compositions characterized in that at least one of the products of formula (I) or (V) as defined in any one of claims 1 to 5 and 7 to 9 or at least one of their addition salts with pharmaceutically acceptable mineral or organic acids is used as active ingredient in a form intended for this use.

11. Preparation process for pharmaceutical compositions characterized in that the product of formula (I) as defined in claim 6 is used as active ingredient in a form intended for this use.

## Patentansprüche (Patentansprüche für folgende(n) Vertragsstaat(en): AT, BE, CH, DE, DK, FR, GB, IT, LI, LU, NL, SE)

1. Verbindungen der Formel (I) 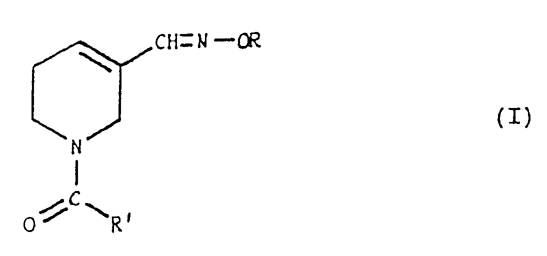 in der
- R ein Wasserstoffatom, einen linearen, verzweigten oder cyclischen, gesättigten oder ungesättigten Alkylrest mit bis zu 8 Kohlenstoffatomen darstellt,
- R' den Rest einer Aminosäure oder eines Peptids mit 2, 3 oder 4 Aminosäuren bedeutet,
- die Gruppe CO von der α-Carboxylgruppe der Aminosäure oder von der CO-Endgruppe des Peptids stammt,
sowie ihre Additionssalze mit organischen Säuren oder Mineralsäuren.

2. Verbindungen der Formel (I) wie in Anspruch 1 definiert, worin R einen linearen, gesättigten oder ungesättigten Alkylrest mit bis zu 4 Kohlenstoffatomen darstellt, sowie ihre Additionssalze mit organischen Säuren oder Mineralsäuren.

3. Verbindungen der Formel (I) wie in Anspruch 2 definiert, worin R einen Methylrest darstellt, sowie ihre Additionssalze mit organischen Säuren oder Mineralsäuren.

4. Verbindungen der Formel (I) wie in irgendeinem der Ansprüche 1 bis 3 definiert, worin R' den Rest einer α-Amino-carbonsäure der Konfiguration L darstellt, sowie ihre Additionssalze mit organischen Säuren oder Mineralsäuren.

5. Verbindungen der Formel (I) wie in irgendeinem der Ansprüche 1 bis 4 definiert, worin R' den Rest von Alanin darstellt, sowie ihre Additionssalze mit organischen Säuren oder Mineralsäuren.

6. Verbindung wie in Anspruch 5 definiert, mit dem folgenden Namen:
- (L-)-2-Amino-1-[3-(methoxyimino)-methyl-1,2,5,6-tetrahydro-1-pyridinyl]-1-propanon.

7. Verbindungen der Formel (I) wie in irgendeinem der Ansprüche 1 bis 4 definiert, worin R' den Rest einer Aminosäure mit zwei Säurefunktionen darstellt, sowie ihre Additionssalze mit organischen Säuren oder Mineralsäuren.

8. Verbindungen der Formel (I) wie in Anspruch 7 definiert, worin R' den Rest der Asparaginsäure darstellt, sowie ihre Additionssalze mit organischen Säuren oder Mineralsäuren.

9. Verbindungen der Formel (V) die in Form der Verbindung der Formel 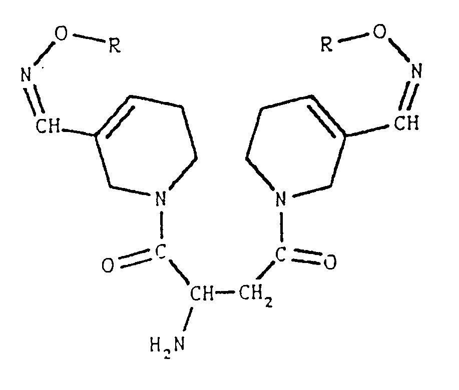 vorliegen, in der R wie in Anspruch 1 definiert ist.

10. Als Medikamente die Verbindungen der Formeln (I) oder (V) wie in irgendeinem der Ansprüche 1 bis 5 und 7 bis 9 definiert, sowie ihre Additionssalze mit pharmazeutisch akzeptablen organischen Säuren oder Mineralsäuren.

11. Als Medikament die Verbindung der Formel (I) wie in Anspruch 6 definiert, sowie ihre Additionssalze mit organischen Säuren oder Mineralsäuren.

12. Pharmazeutische Zusammensetzungen, die als Wirkstoff mindestens ein Medikament wie in Anspruch 10 oder 11 definiert umfassen.

13. Verfahren zur Herstellung der Verbindungen der Formel (I) wie in Anspruch 1 definiert, dadurch gekennzeichnet, daß man eine Säure der Formel (II) 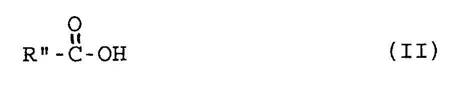 in der R'' den Rest der Aminosäure oder des Peptids R'CO₂H darstellt, worin die Amino-Endgruppe blockiert ist und die Carboxylfunktion aktiviert wurde, mit der Verbindung der Formel (III) 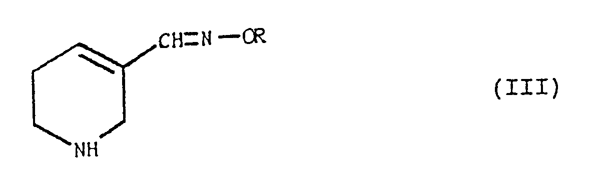 zur Reaktion bringt, in der R seine vorstehende Bedeutung beibehält, um die Verbindung der Formel (I') 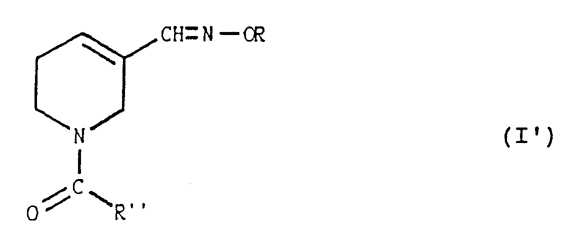 zu erhalten, und man anschließend die Verbindung der Formel (I') der Einwirkung eines Mittels zur Freisetzung der Aminfunktion unterzieht, um die Verbindung der Formel (I) 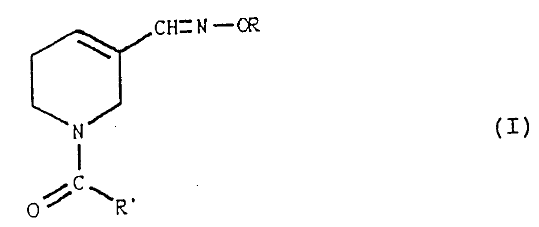 zu erhalten, die man, wenn gewünscht, der Einwirkung einer Säure unterzieht, um das Salz zu bilden.

14. Verfahren nach Anspruch 13 zur Herstellung der Verbindungen der Formel (I) wie in Anspruch 5 definiert, dadurch gekennzeichnet, daß man eine Verbindung der Formel (II_{A}) 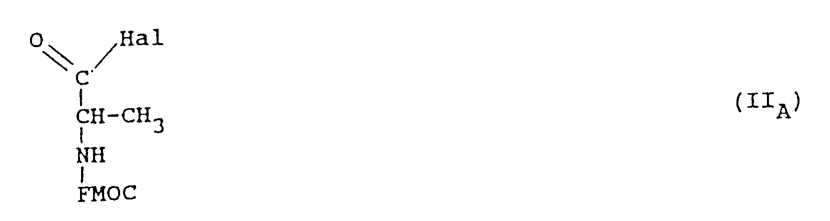 in der Hal ein Halogenatom darstellt und FMOC den Fluorenylmethoxycarbonyl-Rest bedeutet, der Einwirkung einer Verbindung der Formel (III) 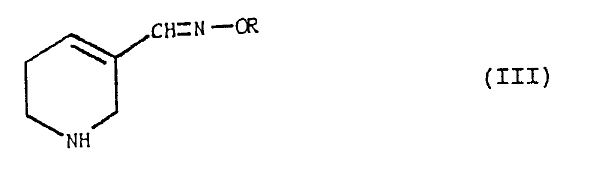 unterzieht, um die Verbindung der Formel (IV) 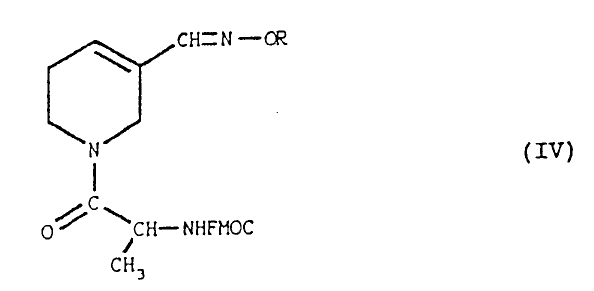 zu erhalten, bei der man die Aminfunktion freisetzt, um die entsprechende Verbindung der Formel (I_{A}) 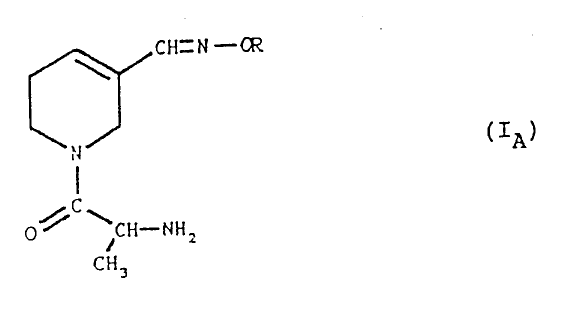 zu erhalten.

## Patentansprüche (Patentansprüche für folgende(n) Vertragsstaat(en): ES)

1. Verfahren zur Herstellung der Verbindungen der Formel (I) 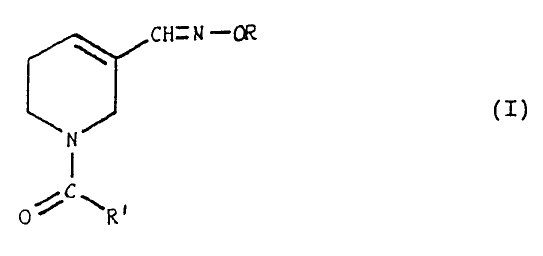 in der
- R ein Wasserstoffatom, einen linearen, verzweigten oder cyclischen, gesättigten oder ungesättigten Alkylrest mit bis zu 8 Kohlenstoffatomen darstellt,
- R' den Rest einer Aminosäure oder eines Peptids mit 2, 3 oder 4 Aminosäuren bedeutet,
- die Gruppe CO von der α-Carboxylgruppe der Aminosäure oder von der CO-Endgruppe des Peptids stammt,
sowie ihrer Additionssalze mit organischen Säuren oder Mineralsäuren, dadurch gekennzeichnet, daß man eine Säure der Formel (II) 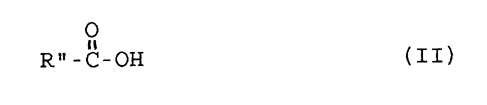 in der R'' den Rest der Aminosäure oder des Peptids R'CO₂H darstellt, worin die Amino-Endgruppe blockiert ist und die Carboxylfunktion aktiviert wurde, mit der Verbindung der Formel (III) 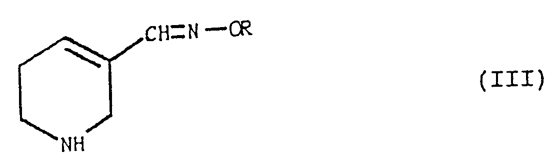 zur Reaktion bringt, in der R seine vorstehende Bedeutung beibehält, um die Verbindung der Formel (I') 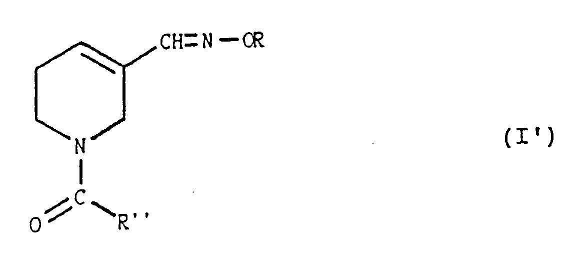 zu erhalten, und man anschließend die Verbindung der Formel (I') der Einwirkung eines Mittels zur Freisetzung der Aminfunktion unterzieht, um die Verbindung der Formel (I) 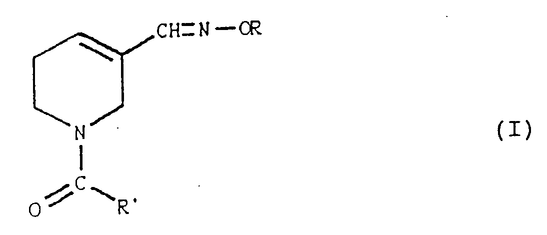 zu erhalten, die man, wenn gewünscht, der Einwirkung einer Säure unterzieht, um das Salz zu bilden.

2. Verfahren nach Anspruch 1, dadurch gekennzeichnet, daß man als Ausgangsprodukt ein Produkt der Formel (III) verwendet, worin R einen linearen, gesättigten oder ungesättigten Alkylrest mit bis zu 4 Kohlenstoffatomen darstellt.

3. Verfahren nach Anspruch 1, dadurch gekennzeichnet, daß man als Ausgangsprodukt ein Produkt der Formel (III) verwendet, worin R einen Methylrest darstellt.

4. Verfahren nach Anspruch 1, dadurch gekennzeichnet, daß man als Ausgangsprodukt ein Produkt der Formel (II) verwendet, worin R'' den Rest einer α-Amino-carbonsäure der Konfiguration L darstellt.

5. Verfahren nach Anspruch 1, dadurch gekennzeichnet, daß man eine Verbindung der Formel (II_{A}) 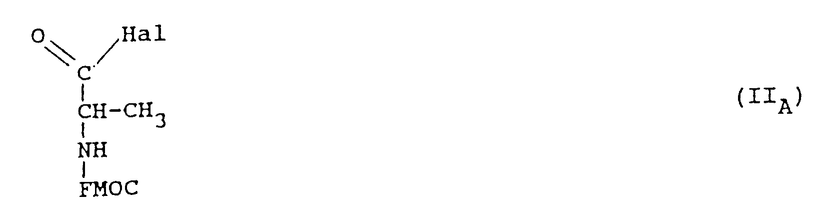 in der Hal ein Halogenatom darstellt und FMOC den Fluorenylmethoxycarbonyl-Rest bedeutet, der Einwirkung einer Verbindung der Formel (III) 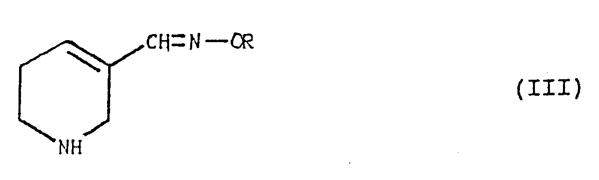 unterzieht, um die Verbindung der Formel (IV) 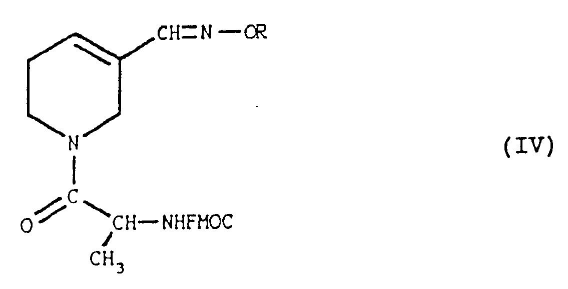 zu erhalten, bei der man die Aminfunktion freisetzt, um die entsprechende Verbindung der Formel (I_{A}) 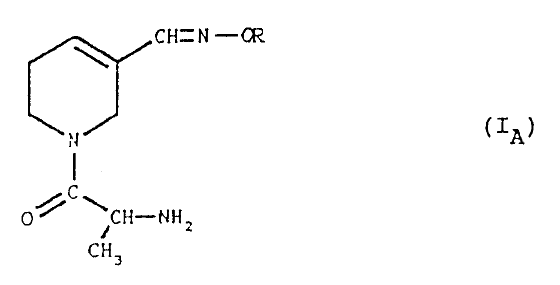 zu erhalten.

6. Verfahren nach Anspruch 5, dadurch gekennzeichnet, daß man die eingesetzten Produkte der Formeln (II_{A}) und (III) in der Weise auswählt, daß man das (L-)-2-Amino-1-[3-(methoxyimino)-methyl-1,2,5,6-tetrahydro-1-pyridinyl]-1-propanon herstellt.

7. Verfahren nach Anspruch 1, dadurch gekennzeichnet, daß man als Ausgangsprodukt ein Produkt der Formel (II) verwendet, worin R'' den Rest einer Aminosäure mit zwei Säurefunktionen darstellt.

8. Verfahren nach Anspruch 7, dadurch gekennzeichnet, daß das als Ausgangsprodukt verwendete Produkt der Formel (II) Asparaginsäure ist.

9. Verfahren, dadurch gekennzeichnet, daß man die eingesetzten Produkte der Formeln (II') und (III) in der Weise auswählt, daß man ein Produkt herstellt, das in Form der Verbindung der Formel (V) 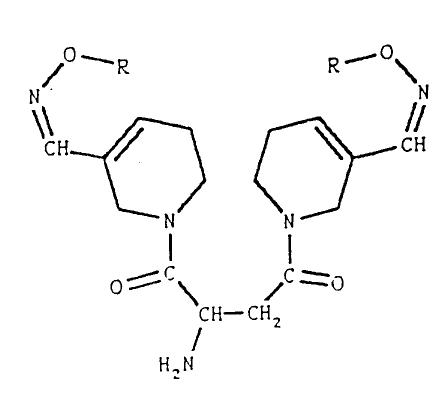 vorliegt, in der R wie in Anspruch 1 definiert ist.

## Patentansprüche (Patentansprüche für folgende(n) Vertragsstaat(en): GR)

1. Verfahren zur Herstellung der Verbindungen der Formel (I) 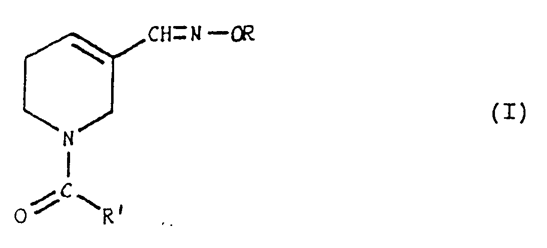 in der
- R ein Wasserstoffatom, einen linearen, verzweigten oder cyclischen, gesättigten oder ungesättigten Alkylrest mit bis zu 8 Kohlenstoffatomen darstellt,
- R' den Rest einer Aminosäure oder eines Peptids mit 2, 3 oder 4 Aminosäuren bedeutet,
- die Gruppe CO von der α-Carboxylgruppe der Aminosäure oder von der CO-Endgruppe des Peptids stammt,
sowie ihrer Additionssalze mit organischen Säuren oder Mineralsäuren, dadurch gekennzeichnet, daß man eine Säure der Formel (II) 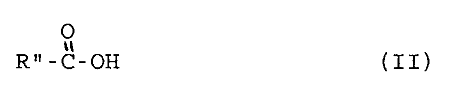 in der R'' den Rest der Aminosäure oder des Peptids R'CO₂H darstellt, worin die Amino-Endgruppe blockiert ist und die Carboxylfunktion aktiviert wurde, mit der Verbindung der Formel (III) 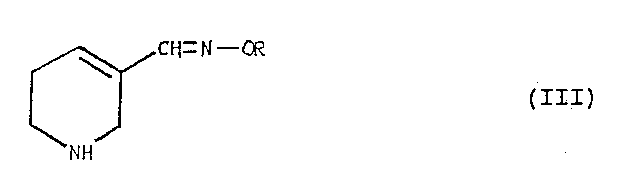 zur Reaktion bringt, in der R seine vorstehende Bedeutung beibehält, um die Verbindung der Formel (I') 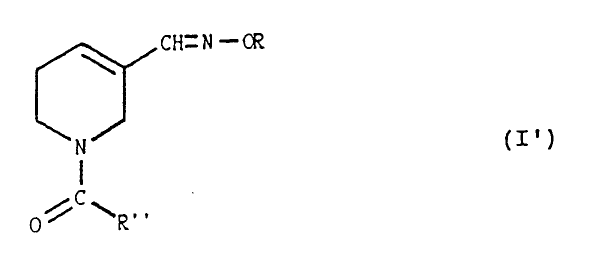 zu erhalten, und man anschließend die Verbindung der Formel (I') der Einwirkung eines Mittels zur Freisetzung der Aminfunktion unterzieht, um die Verbindung der Formel (I) 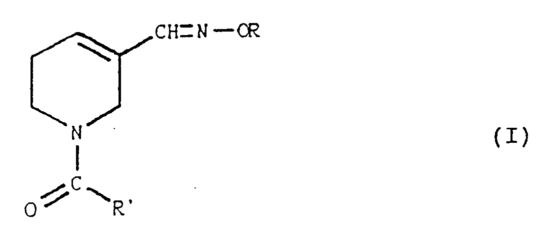 zu erhalten, die man, wenn gewünscht, der Einwirkung einer Säure unterzieht, um das Salz zu bilden.

2. Verfahren nach Anspruch 1, dadurch gekennzeichnet, daß man als Ausgangsprodukt ein Produkt der Formel (III) verwendet, worin R einen linearen, gesättigten oder ungesättigten Alkylrest mit bis zu 4 Kohlenstoffatomen darstellt.

3. Verfahren nach Anspruch 1, dadurch gekennzeichnet, daß man als Ausgangsprodukt ein Produkt der Formel (III) verwendet, worin R einen Methylrest darstellt.

4. Verfahren nach Anspruch 1, dadurch gekennzeichnet, daß man als Ausgangsprodukt ein Produkt der Formel (II) verwendet, worin R'' den Rest einer α-Amino-carbonsäure der Konfiguration L darstellt.

5. Verfahren nach Anspruch 1, dadurch gekennzeichnet, daß man eine Verbindung der Formel (II_{A}) 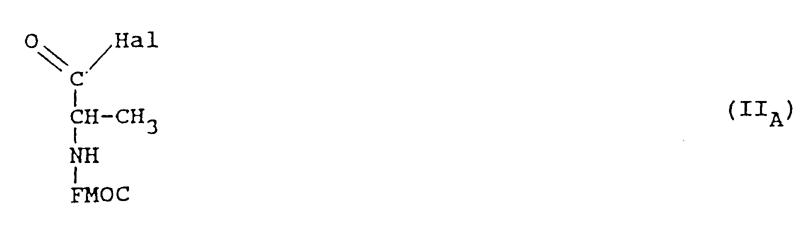 in der Hal ein Halogenatom darstellt und FMOC den Fluorenylmethoxycarbonyl-Rest bedeutet, der Einwirkung einer Verbindung der Formel (III) 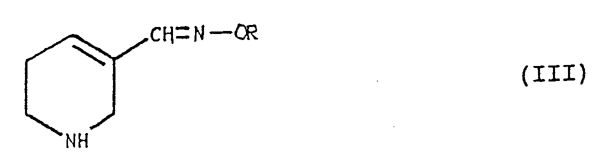 unterzieht, um die Verbindung der Formel (IV) 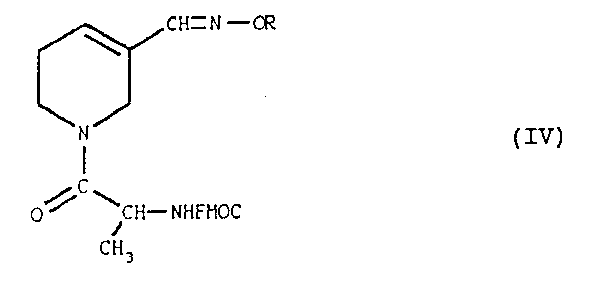 zu erhalten, bei der man die Aminfunktion freisetzt, um die entsprechende Verbindung der Formel (I_{A}) 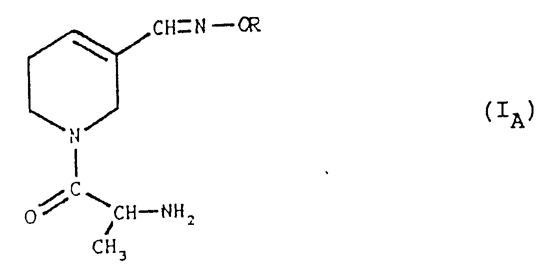 zu erhalten.

6. Verfahren nach Anspruch 5, dadurch gekennzeichnet, daß man die eingesetzten Produkte der Formeln (II_{A}) und (III) in der Weise auswählt, daß man das (L-)-2-Amino-1-[3-(methoxyimino)-methyl-1,2,5,6-tetrahydro-1-pyridinyl]-1-propanon herstellt.

7. Verfahren nach Anspruch 1, dadurch gekennzeichnet, daß man als Ausgangsprodukt ein Produkt der Formel (II) verwendet, worin R'' den Rest einer Aminosäure mit zwei Säurefunktionen darstellt.

8. Verfahren nach Anspruch 7, dadurch gekennzeichnet, daß das als Ausgangsprodukt verwendete Produkt der Formel (II) Asparaginsäure ist.

9. Verfahren, dadurch gekennzeichnet, daß man die eingesetzten Produkte der Formeln (II') und (III) in der Weise auswählt, daß man ein Produkt herstellt, das in Form der Verbindung der Formel (V) 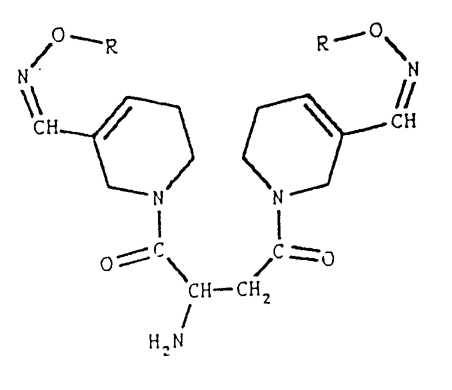 vorliegt, in der R wie in Anspruch 1 definiert ist.

10. Verfahren zur Herstellung von pharmazeutischen Zusammensetzungen, dadurch gekennzeichnet, daß man als Wirkstoff mindestens eines der Produkte der Formeln (I) oder (V), wie in irgendeinem der Ansprüche 1 bis 5 und 7 bis 9 definiert, verwendet oder mindestens eines ihrer pharmazeutisch akzeptablen Additionssalze mit organischen Säuren oder Mineralsäuren in einer für diesen Verwendungszweck vorgesehenen Form.

11. Verfahren zur Herstellung von pharmazeutischen Zusammensetzungen, dadurch gekennzeichnet, daß man als Wirkstoff das Produkt der Formel (I) wie in Anspruch 6 definiert verwendet, in einer für diesen Verwendungszweck vorgesehenen Form.
